**Europäisches Patentamt**

**European Patent Office**

**Office européen des brevets**

(19)

(11) Veröffentlichungsnummer: **0 115 336**
**A2**

(12) # EUROPÄISCHE PATENTANMELDUNG

(21) Anmeldenummer: 84100781.8

(22) Anmeldetag: 25.01.84

(51) Int. Cl.³: **A 61 B 3/04**

(30) Priorität: 25.01.83 DE 3302355

(43) Veröffentlichungstag der Anmeldung:
08.08.84 Patentblatt 84/32

(84) Benannte Vertragsstaaten:
AT CH DE FR GB IT LI

(71) Anmelder: Nickel, Franz, Dr.
Neufeldstrasse 42 R
D-8037 Olching(DE)

(72) Erfinder: Nickel, Franz, Dr.
Neufeldstrasse 42 R
D-8037 Olching(DE)

(74) Vertreter: Schiller, Walter, Dr.
Willibaldstrasse 36
D-8000 München 21(DE)

(54) **Verfahren und Vorrichtung zur Refraktionsbestimmung.**

(57) Beschrieben wird ein Verfahren und eine Vorrichtung zur Refraktionsbestimmung mit Meßgläsern, die in eine Fassung eingesetzt werden. Dabei wird als Probierfassung die von der zu untersuchenden Person für die spätere Brille ausgewählte Fassung (1) verwendet. In die Fassung werden Scheiben (2) eingesetzt oder an der Fassung angebracht. Zentrisch zu den Durchblickpunkten der zu untersuchenden Person werden Aufnahmen (4) für Gläser mit optischer Wirkung angebracht. Hierauf wird die Refraktion auf an sich bekannte Weise ausgeführt.

FIG. 2

EP 0 115 336 A2

DIPL.-PHYSIKER     **DR. WILHELM MÜNICH**     0115336 WALT

**DR. WALTER SCHILLER**     RECHTSANWALT

DR. MÜNICH · WILLIBALDSTR. 36 · D 8000 MÜNCHEN 21     TEL.: 089/5808049 · TELEX: 528464

MEIN ZEICHEN: (BITTE BEI
RÜCKANTWORT ANGEBEN):

Dr. Franz Nickel
Neufeldstr. 42 R

8037 Olching

---

Verfahren und Vorrichtung zur Refraktionsbestimmung

---

## Beschreibung

Die Erfindung bezieht sich auf ein Verfahren und eine Vorrichtung zur Refraktionsbestimmung gemäß dem Oberbegriff der Patentansprüche 1 bzw. 10.

Bei einem bekannten Verfahren zur Refraktionsbestimmung mit Meßgläsern wird eine spezielle Probierfassung verwendet. In diese Probierfassung werden ein oder mehrere Meßgläser zur Bestimmung der Fehlsichtigkeit eingesetzt. Nachteilig bei diesem bekannten Verfahren ist jedoch, daß sich der Abstand Hornhaut/Scheitel des Refraktions-Meßglases bei der Probierfassung in den meisten Fällen von dem Abstand Hornhaut/Scheitel des

BANKVERBINDUNG: STADTSPARKASSE MÜNCHEN (BLZ 701 50000) 38-125514 ·
KREISSPARKASSE MÜNCHEN (BLZ 702 50150) 806174 · POSTSCHECKAMT MÜNCHEN 150505-802

Brillenglases bei der von der zu untersuchenden Person (Klient) später getragenen Brille unterscheidet.

Der Erfindung liegt die Aufgabe zugrunde, ein Verfahren und eine Vorrichtung anzugeben, die die Bestimmung der zum Ausgleich der Fehlsichtigkeit erforderlichen Korrekturgläser unabhängig von Meßunsicherheiten bei der Bestimmung des Hornhaut/Scheitel-Abstandes erlauben.

Diese Aufgabe wird erfindungsgemäß durch die im kennzeichnenden Teil der Patentansprüche 1 bzw. 9 angegebenen Merkmale gelöst.

Durch die Verwendung der für die spätere Brille ausgewählten Fassung als Probierfassung wird erreicht, daß der Hornhaut/Scheitel-Abstand der Probiergläser gleich dem Abstand bei der späteren Brille ist, oder mit diesem Abstand in einer leicht zu ermittelnden Beziehung steht.

Insbesondere ist es gemäß Anspruch 2 leicht möglich, den tatsächlich erforderlichen Brechwert aus dem Brechwert der in die Aufnahmen eingesetzten Meßgläser und der Abstandsdifferenz der Meßgläser und der späteren Brillengläser von der Hornhaut zu bestimmen.

Weitere Ausgestaltungen der Erfindung sind in den Ansprüchen 3 f. angegeben.

Die Verwendung eines Scheitelbrechwert-Meßgerätes zum Bestimmen des Gesamt-Brechwertes der in die Aufnahmen eingesetzten Gläser gemäß Anspruch 3 ist der einfachen Addition der Einzelbrechwerte vorzuziehen.

Die Befestigung der Aufnahmen in Löchern der Anpaßscheiben erlaubt es, die Gläser mit optischer Wirkung möglichst nahe an die Ebene des späteren Brillenglases zu rücken (Anspruch 4 bzw. 14).

Dieser Vorteil wird weiter vergrößert, wenn der Hauptanteil der Fehlsichtigkeit mit einem Glas ausgeglichen wird, das sich in einer genau definierten Lage und möglichst nahe der Ebene der Brillengläser befindet (Anspruch 13 in Verbindung mit Anspruch 14).

Natürlich ist es auch möglich, die Aufnahmen gemäß Anspruch 5 bzw. 17 an den Scheiben anzukleben. Dies kann insbesondere dann erfolgen, wenn als Probierfassung eine Fassung verwendet wird, die von der zu untersuchenden Person bereits getragen worden ist, und lediglich eine Überrefraktionsbestimmung erfolgen soll (Anspruch 8). Hierbei wird die bisherige Brille der zu untersuchenden Person – sofern sie weiter verwendet werden soll – benutzt. An den Brillengläsern werden die Aufnahmen für die Refraktions-Meßgläser angeklebt und eine sogenannte Überrefraktionsbestimmung ausgeführt, d.h. es wird die erforderliche optische Zusatzwirkung bestimmt.

Die Drehbarkeit der Aufnahmen gemäß Anspruch 6 bzw. 18 erlaubt beispielsweise die Bestimmung der Astigmatismus-Achse.

Die Erfindung wird nachstehend anhand eines Ausführungsbeispiels unter Bezugnahme auf die Zeichnung näher beschrieben. Es zeigen:

Fig. 1 eine Brillenfassung mit eingesetzten Anpaßscheiben,

Fig. 2 und 3 eine Brillenfassung mit angebrachten Aufnahmen in Aufsicht bzw. im Schnitt, und

Fig. 4 eine Winkelmeßeinrichtung für die Winkelstellung der Gläser mit optischer Wirkung.

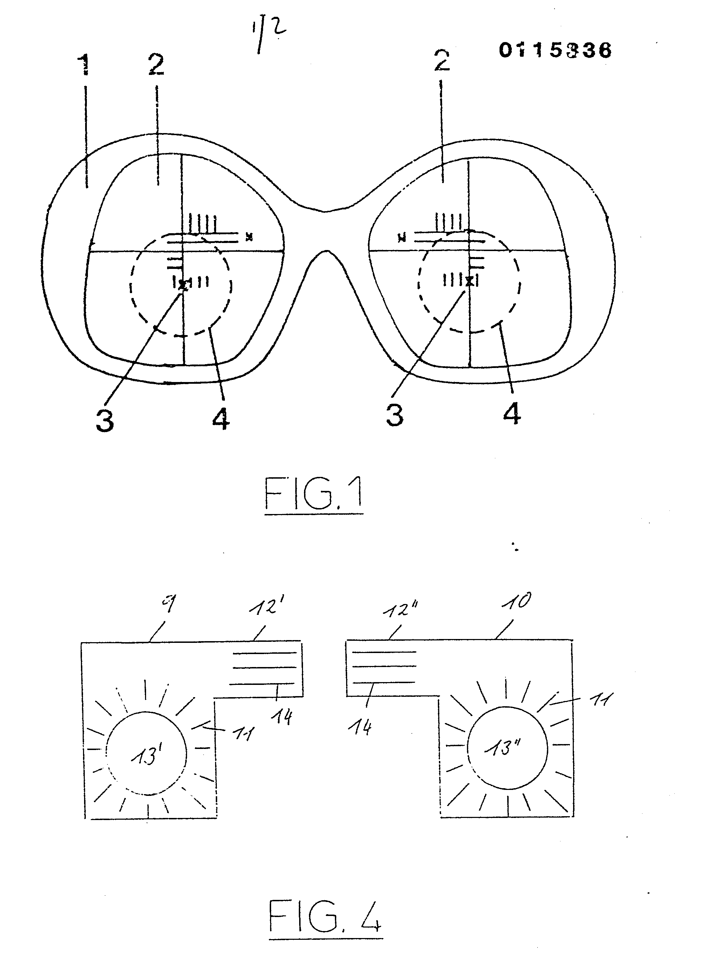

Fig. 1 zeigt eine Brillenfassung 1, in die Anpaßscheiben 2 aus einem durchsichtigen Kunststoffmaterial eingesetzt sind. Auf den Anpaßscheiben 2 sind die Durchblickpunkte 3 der zu untersuchenden Person markiert. Zentrisch zu den Durchblickpunkten 3 sind Löcher 4 in die Anpaßscheiben 3 eingestanzt.

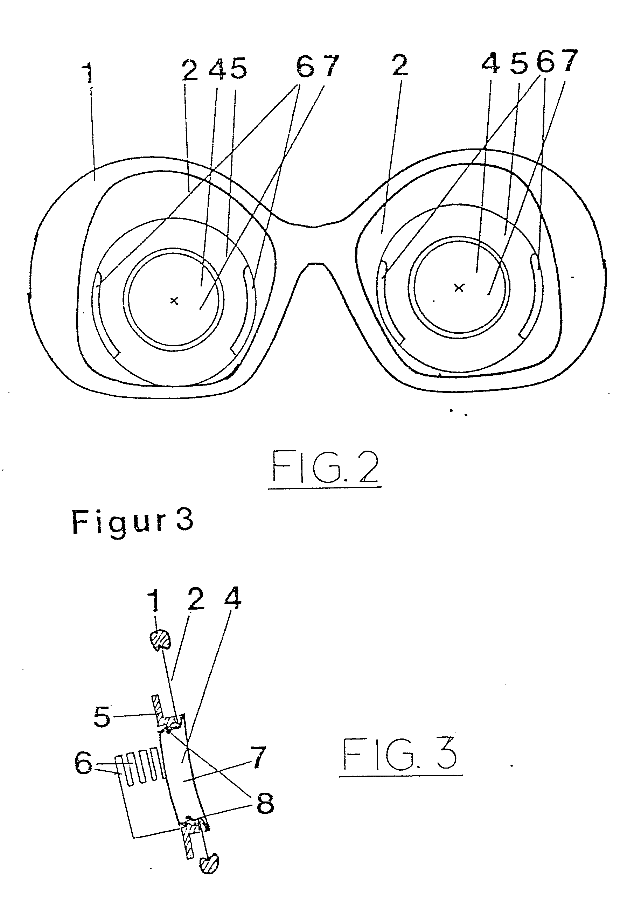

Fig. 2 und 3 zeigen in die Löcher 4 eingesetzte Aufnahmen 5 für nicht dargestellte Refraktions-Meßglä - ser. In die Aufnahmen können ein oder mehrere Refraktions-Meßgläser eingesetzt werden, die von Federn 6 gehalten werden.

Die Aufnahmen 5 können auch ein Spritzgußteil oder ein sonstiges Kunststoffteil mit "gewellten" Seiten sein, in deren "Vertiefungen" die Meßgläser eingesetzt werden. In diesem Falle ist es möglich, auf die Federn zu verzichten, da die Meßgläser durch Klemmung gehalten werden.

Die Aufnahmen 5 können durch einen mit einer Stufe versehenen Gegenring, der von der augenseitigen Fläche der Anpaßscheibe 2 in die Aufnahme eingesteckt wird, gehalten werden. In diesen Gegenring kann ein Refraktions-Meßglas zum Ausgleich des Hauptanteils der Fehlsichtigkeit eingelegt werden. Besonders vorteilhaft ist die dargestellte Ausführungsform, bei der ein Refraktions-Meßglas 7 am Umfangsrand mit einer Stufe 8 versehen ist. Dieses Meßglas 7 wird von der augenseitigen Fläche der Anpaßscheibe 2 in die Aufnahme 5 eingesteckt. Da die Befestigung lediglich durch eine Steckverbindung und Klemmung an der Anpaßscheibe erfolgt, kann die Aufnahme gegenüber der Anpaßscheibe beispielsweise zur Bestimmung der Lage einer Zylinderachse gedreht werden.

Wenn Meßgläser verwendet werden, die zur Bestimmung der Zylinderachse in der Aufnahme gedreht werden sollen, kann die Aufnahme auch gegen die Anpaßscheibe drehfest festgelegt werden. Hierzu können beispielsweise Löcher am Umfang des Lochs 4 in die Anpaßscheibe 2 gestanzt werden, durch die sich Zapfen an der Aufnahme erstrecken.

Sowohl in dem Fall, daß die Aufnahmen gedreht werden, als auch in dem Fall, daß die Meßgläser gedreht werden, ist es vorteilhaft, wenn eine Winkelmeßeinrichtung zur Bestimmung der Winkellage der Gläser mit optischer Wirkung verwendet wird.

Fig. 4 zeigt eine derartige Winkelmeßeinrichtung. Die Winkelmeßeinrichtung 9 wird an der Aufnahme 5 für das rechte Auge (links in Fig. 2) und die Winkelmeßeinrichtung 10 an der Aufnahme für das linke Auge befestigt. Diese Befestigung kann beispielsweise dadurch erfolgen, daß die Winkelmeßeinrichtungen auf die Scheiben gelegt und anschließend die Aufnahme in die Löcher 4 in den Scheiben 3 eingesteckt werden. Hierzu weisen die Winkelmeßeinrichtungen Löcher 13' und 13" auf.

Die Winkelmeßeinrichtungen tragen Winkelskalen 11, die bezüglich der Brillenfassung 1 dadurch ausgerichtet werden, daß ihre Fortsätze 12' bzw. 12" miteinander fluchtend verbunden werden. Die aufgedruckten Striche 13 dienen dabei zur Ausrichtung der Fortsätze.

Bei einer weiteren - nicht dargestellten - Ausführungsform der Erfindung wird die Aufnahme an der Anpaßscheibe angeklebt. Dabei kann die Aufnahme beispielsweise zweiteilig ausgebildet und der Teil der Aufnahme, in den die Meßgläser eingesetzt werden, gegenüber dem angeklebten Teil drehbar sein.

Die Erfindung hat den Vorteil, daß sich die Meßgläser bei der Refraktionsbestimmung in einer genau definierten Lage zu der Lage des späteren Brillenglases befinden. Insbesondere wenn das Meßglas 7 zum Ausgleich des Hauptanteils der Fehlsichtigkeit verwendet wird, sind nur noch geringe Korrekturen bei der Bestimmung des tatsächlich erforderlichen Brechwerts erforderlich, da der Hornhaut/Scheitel-Abstand des Refraktions-Meßglases 7, das in die Aufnahme eingesteckt wird, sich kaum von dem Hornhaut/Scheitel-Abstand bei der späteren Brille unterscheidet.

Patentansprüche

1. Verfahren zur Refraktionsbestimmung mit Meßgläsern, die in eine Fassung eingesetzt werden, g e k e n n z e i c h n e t durch die Kombination folgender Schritte:

(A) als Probierfassung wird die von der zu untersuchenden Person für die spätere Brille ausgewählte Fassung verwendet,

(B) in die Fassung werden Scheiben eingesetzt oder an der Fassung angebracht,

(C) der Durchblickpunkt der Person wird markiert,

(D) an den Anpaßscheiben werden zentrisch zu den markierten Durchblickpunkten Aufnahmen für Gläser mit optischer Wirkung angebracht,

(E) die Refraktion wird auf an sich bekannte Weise ausgeführt.

2. Verfahren nach Anspruch 1, dadurch gekennzeichnet, daß der bei der zu untersuchenden Person tatsächlich erforderliche Brechwert aus dem Brechwert der in die Aufnahmen eingesetzten Gläser und dem Abstand der Gläser von der Ebene der Fassung berechnet wird.

3. Verfahren nach Anspruch 1 oder 2, dadurch gekennzeichnet, daß der Brechwert der in die Aufnahmen eingesetzten Gläser mit einem Scheitelbrechwert-Meßgerät bestimmt wird.

4. Verfahren nach einem der Ansprüche 1 bis 3, dadurch gekennzeichnet, daß zur Befestigung der Aufnahmen Anpaßscheiben verwendet werden, in die zentrisch zu den Durchblickpunkten Löcher gestanzt werden, in die die Anpaßscheiben eingesetzt werden.

5. Verfahren nach einem der Ansprüche 1 bis 3, dadurch gekennzeichnet, daß zur Befestigung der Aufnahmen Anpaßscheiben verwendet werden, an denen die Aufnahmen angeklebt werden.

6. Verfahren nach einem der Ansprüche 1 bis 5, dadurch gekennzeichnet, daß die Aufnahmen gegenüber der Fassung drehbar sind.

7. Verfahren nach einem der Ansprüche 1 bis 6, dadurch gekennzeichnet, daß die Aufnahmen gegenüber dem Durchblickpunkt verschiebbar sind.

8. Verwendung des Verfahrens nach einem der Ansprüche 1 bis 7 zur sogenannten Überrefraktionsbestimmung, wobei die Aufnahmen an den bestehenden Korrektionsgläsern angebracht werden.

9. Vorrichtung zur Refraktionsbestimmung mit Gläsern mit optischer Wirkung, die in eine Fassung einsetzbar sind, gekennzeichnet durch mindestens eine Aufnahme (5) für die Meßgläser, die zentrisch zum Durchblickpunkt (3) der zu untersuchenden Person an Scheiben (2) der für die spätere Brille verwendeten Fassung (1) angebracht ist.

10. Vorrichtung nach Anspruch 9, dadurch gekennzeichnet, daß die Scheiben sog. Anpaßscheiben sind.

11. Vorrichtung nach Anspruch 9 oder 10, dadurch gekennzeichnet, daß an den Aufnahmen eine Winkelmeßeinrichtung für die Winkelstellung der Gläser mit optischer Wirkung anbringbar ist.

0115336

12. Vorrichtung nach einem der Ansprüche 9 bis 11, dadurch gekennzeichnet, daß die Gläser mit optischer Wirkung an sich bekannte Meßgläser sein können.

13. Vorrichtung nach einem der Ansprüche 9 bis 12, dadurch gekennzeichnet, daß der Hauptanteil der Fehlsichtigkeit mit einem Glas ausgeglichen wird, das sich in einer genau definierten Lage bezüglich der Ebene der Brillengläser befindet.

14. Vorrichtung nach einem der Ansprüche 9 bis 13, dadurch gekennzeichnet, daß die Aufnahmen in Löcher (4) einsetzbar sind, die zentrisch zu den Durchblickpunkten in die Scheiben eingestanzt sind.

15. Vorrichtung nach Anspruch 14, dadurch gekennzeichnet, daß ein von der augenseitigen Fläche der Scheibe in die Aufnahme (5) eingestecktes Klemmelement (7) die Aufnahme an der Scheibe hält.

16. Vorrichtung nach Anspruch 15, dadurch gekennzeichnet, daß als Klemmelement ein Glas (7) mit optischer Wirkung verwendet wird, das zum Ausgleich des Hauptanteils der Fehlsichtigkeit dient.

17. Vorrichtung nach einem der Ansprüche 9 bis 16, dadurch gekennzeichnet, daß die Aufnahmen (5) an den Scheiben anklebbar sind.

18. Vorrichtung nach einem der Ansprüche 9 bis 17, dadurch gekennzeichnet, daß die Aufnahmen (5) gegenüber der Fassung drehbar sind.

19. Vorrichtung nach einem der Ansprüche 9 bis 18, dadurch gekennzeichnet, daß die Aufnahmen (5) gegenüber dem Durchblickpunkt (3) verschiebbar sind.

0115336

20. Vorrichtung nach einem der Ansprüche 9 bis 19, dadurch gekennzeichnet, daß die Aufnahmen ein Spritzgußteil sind.

0115336

FIG.1

FIG. 4

1   2 45   67   2   4 5 67

## FIG.2

Figur 3

1 2 4

5

6   7

8

## FIG. 3